# EUROPÄISCHE PATENTSCHRIFT

(11) **EP 0 527 380 B1**
(45) Veröffentlichungstag und Bekanntmachung des Hinweises auf die Patenterteilung: **03.01.1996**
(21) Anmeldenummer: 92112780.9
(22) Anmeldetag: 27.07.1992
(51) Int. Cl.: C07D 239/60, C07D 251/30, A01N 43/54

(54) **Salicylsäurederivate als selektive Herbizide**
Salicylic acid derivatives, as selective herbicides
Dérivés d'acide salicylique utiles comme herbicides sélectifs

(30) Priorität: 10.08.1991 DE 4126936
(43) Veröffentlichungstag der Anmeldung: 17.02.1993
(73) Patentinhaber: BASF Aktiengesellschaft, D-67063 Ludwigshafen (DE)
(72) Erfinder: Vogelbacher, Uwe Josef, Dr., W-6700 Ludwigshafen (DE); Rheinheimer, Joachim, Dr., W-6700 Ludwigshafen (DE); Saupe, Thomas, Dr., W-6902 Sandhausen (DE); Meyer, Norbert, Dr., W-6802 Ladenburg (DE); Gerber, Matthias, Dr., W-6704 Mutterstadt (DE); Westphalen, Karl-Otto, Dr., W-6720 Speyer (DE); Walter, Helmut, Dr., W-6719 Obrigheim (DE)

(56) Entgegenhaltungen:
- EP-A- 0 402 751
- EP-A- 0 426 476

## Beschreibung

Die vorliegende Erfindung betrifft Salicylsäurederivate und deren Schwefelanaloga der Formel I, 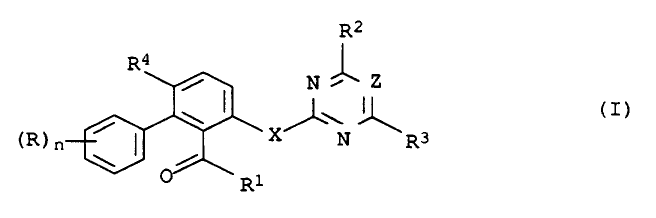
in der die Substituenten folgende Bedeutung haben:
- R: Halogen, C₁-C₄-Alkyl, C₁-C₄-Halogenalkyl, C₁-C₄-Alkoxy, C₁-C₄-Halogenalkoxy und/oder C₁-C₄-Alkylthio;
- n: eine Zahl von 1 bis 3, im Falle von Halogen als Substituenten eine Zahl von 1 bis 5
- R¹: ein Rest 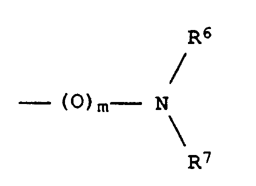 in dem m für 0 und 1 steht und R⁶ und R⁷ die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, bis-C₁-C₆-Dialkylamino, C₃-C₆-Cycloalkyl, gegebenenfalls substituiertes Phenyl;
gegebenenfalls substituiertes C₃-C₆-Cycloalkyl;
gegebenenfalls substituiertes Phenyl;
oder R⁶ zusammen mit R⁷ eine gegebenenfalls substituierte C₄-C₇-Alkylenkette bildet, in der eine CH₂-Gruppe durch Sauerstoff, Schwefel oder -NH ersetzt sein kann;
oder R¹ eine Gruppe 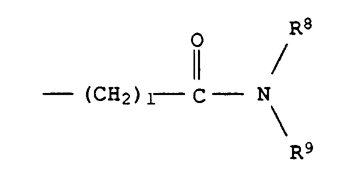 in der R⁸ und R⁹ für Wasserstoff, C₁-C₆-Alkyl, C₃-C₆-Alkenyl oder C₃-C₆-Alkinyl stehen und l die Werte 1, 2, 3 oder 4 annimmt;
oder eine Gruppe

-(CH₂)ₗ-S(=O)ₖ-R¹⁰

in der R¹⁰ für C₁-C₆-Alkyl, C₃-C₆-Alkenyl oder C₃-C₆-Alkinyl, l für 1, 2, 3 oder 4 und k für 0, 1 oder 2 stehen;
oder R¹ ein Rest

-HN-SO₂-R¹¹,

in dem R¹¹ für die Reste C₁-C₆-Alkyl oder Phenyl steht, die ihrerseits ein bis vier der folgenden Substituenten tragen können: Halogen, Nitro, Cyano, C₁-C₆-Alkyl;
ein Rest OR⁵, worin
- R⁵: einen über ein Stickstoffatom gebundenen gegebenenfalls substituierten 5-gliedrigen aromatischen Heterocyclus mit ein bis vier Stickstoffatomen im Ring;
oder einen Rest 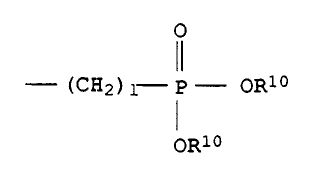 in dem R¹⁰ und l die oben genannte Bedeutung haben, darstellt;
wobei der Ausdruck gegebenenfalls substituiert in den voranstehenden Definitionen bedeutet, daß die so bezeichneten Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio;
- R², R³: C₁-C₄-Alkyl, C₁-C₄-Halogenalkyl, C₁-C₄-Alkoxy, C₁-C₄-Halogenalkoxy und/oder C₁-C₄-Alkylthio;
- R⁴: Wasserstoff, Halogen, Nitro, C₁-C₄-Alkyl, Cyano oder C₁-C₄-Halogenalkyl;
- X: ein Sauerstoff- oder Schwefelatom;
- Z: Stickstoff oder die Methingruppe.

In EP-A 426 476 wird ein herbizid wirksames Salicylsäurederivat mit einem Phenylsubstituenten in 6-Stellung der Struktur A beschrieben. 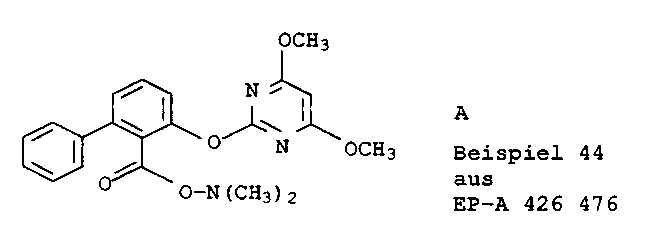
Die Selektivität dieser Verbindung ist jedoch nicht immer befriedigend, insbesondere keineswegs ausreichend bei Applikation in Getreidekulturen. Herbizid wirksame Salicylsäurederivate sind ferner in EP-A 402 751, EP-A 346 789, EP-A 223 406, EP-A 249 708, EP-A 287 072 und EP-A 287 079 beschrieben.

Der Erfindung lag die Aufgabe zugrunde, im Pflanzenschutz gut wirksame Salicyclsäurederivate zur Verfügung zu stellen, die eine deutlich bessere Selektivität, insbesondere in Getreidekulturen aufweisen als strukturell ähnliche Verbindungen des Standes der Technik.

Die vorliegende Erfindung beschreibt nun neue Salicylsäurederivate bzw. deren Schwefelanaloge mit einem substituierten Phenylring in 6-Stellung, wie eingangs definiert, die sich durch eine hervorragende Selektivität in Getreidekulturen gegenüber den bisher beschriebenen Verbindungen gleichen Typs auszeichnen und zudem noch pflanzenwachstumsregulierende, fungizide und/oder nitrifikationshemmende Eigenschaften aufweisen.

Die Herstellung der erfindungsgemäßen Verbindungen erfolgt wie in EP-A 426 476 beschrieben, entweder durch Überführung einer Säure der Formel B (Darstellung in EP-A 402 751 beschrieben) 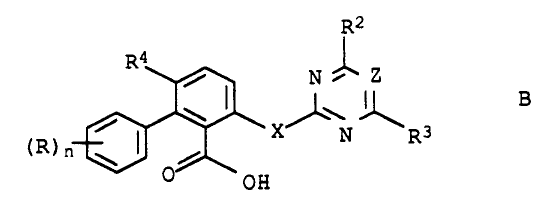
in eine aktivierte Form und anschließende Umsetzung mit einem Hydroxylaminderivat der Formel HO-NR⁶R⁷, einer Verbindung HOR⁵ oder einem Sulfonamid der Formel H₂N-SO₂-R¹¹ wie oben beschrieben, oder durch Überführung einer Salicylsäure der Formel C (Darstellung in EP-A 402 751 beschrieben) 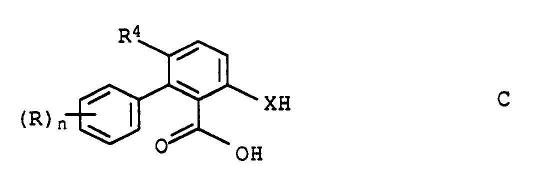
in eine aktivierte Form, Umsetzung mit einem Hydroxylaminderivat der Formel HO-NR⁶R⁷, einer Verbindung HOR⁵ oder einem Sulfonamid der Formel H₂N-SO₂-R¹¹ wie oben beschrieben zu einem Derivat der Formel D 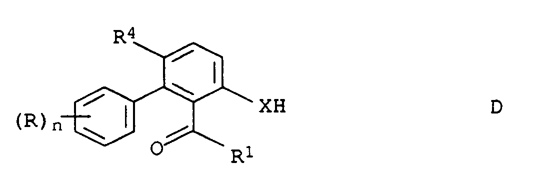
und anschließende Umsetzung mit einem Pyrimidin oder Triazin der Formel E 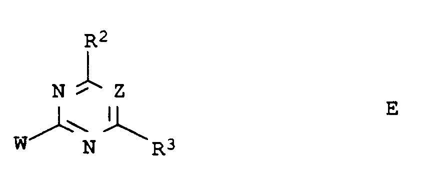
in der W für eine nucleofuge Gruppe wie Chlor, Methylsulfonyl oder Phenylsulfonyl steht, unter Zuhilfenahme einer anorganischen oder organischen Base.

Im Hinblick auf die biologische Wirksamkeit sind Verbindungen I bevorzugt, in denen die Substituenten die folgende Bedeutung haben:
- R: Halogen wie Fluor, Chlor, Brom, Iod, insbesondere Chlor und Brom;
C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl;
C₁-C₄-Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl;
C₁-C₄-Alkoxy wie Methoxy, Ethoxy, 1-Methylethoxy;
C₁-C₄-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy; und/oder
C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;
- n: eine Zahl 1, 2 oder 3, im Fall von R=Halogen auch 4 oder 5;
- R¹: ein Rest 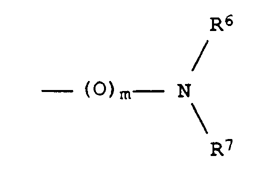 in dem m 0 oder 1 bedeutet und R⁶ und R⁷ gleich oder verschieden sind und folgende Bedeutung haben:
Wasserstoff;
C₁-C₆-Alkyl wie Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl;
C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl,2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;
C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl,
wobei diese Alkyl-, Alkenyl- und Alkinylgruppen ein bis fünf der vorstehend im allgemeinen und im besonderen genannten Halogenatome tragen können, und/oder ein bis zwei der folgenden Gruppen:
Cyano;
Alkoxy mit ein bis vier Kohlenstoffatomen, insbesondere Alkoxy, wie z.B. Methoxy, Ethoxy, Propoxy, 1-Methylethoxy, Butoxy, 1-Methylpropoxy, 2-Methylpropoxy, 1,1-Dimethylethoxy, vorzugsweise Methoxy, Ethoxy und 1-Methylethoxy;
Alkenyloxy mit ein bis vier Kohlenstoffatomen, insbesondere Alkenyloxy, wie z.B. Ethenyloxy, Propenyloxy, 1-Methylethenyloxy, Butenyloxy, 1-Methylpropenyloxy, 2-Methylpropenyloxy, 1,1-Dimethylethenyloxy, vorzugsweise Ethenyloxy und 1-Methylethenyloxy;
Alkinyloxy mit ein bis vier Kohlenstoffatomen, insbesondere Alkinyloxy, wie z.B. Ethinyloxy, Propinyloxy, 1-Methylethinyloxy, Butinyloxy, 1-Methylpropinyloxy, 2-Methylpropinyloxy, 1,1-Dimethylethinyloxy, vorzugsweise Ethinyloxy und 1-Methylethinyloxy;
Alkylthio mit ein bis vier Kohlenstoffatomen, insbesondere Methylthio, Ethylthio, Propylthio, 1-Methylethylthio, Butylthio, 1-Methylpropylthio, 2-Methylpropylthio, 1,1-Dimethylethylthio, vorzugsweise Methylthio, Ethylthio und 1-Methylethylthio;
Alkenylthio mit ein bis vier Kohlenstoffatomen, insbesondere Alkenylthio, wie z.B. Ethenylthio, Propenylthio, 1-Methylethenylthio, Butenylthio, 1-Methylpropenylthio, 2-Methylpropenylthio, 1,1-Dimethylethenylthio, vorzugsweise Ethenylthio und 1-Methylethenylthio;
Alkinylthio mit ein bis vier Kohlenstoffatomen, insbesondere Alkinylthio, wie z.B. Ethinylthio, Propinylthio, 1-Methylethinylthio, Butinylthio, 1-Methylpropinylthio, 2-Methylpropinylthio, 1,1-Dimethylethinylthio, vorzugsweise Ethinylthio und 1-Methylethinylthio;
Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy, Pentafluorethoxy, insbesondere Difluormethoxy und Trifluormethoxy;
Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 1,1-Dimethylethylcarbonyl, Pentylcarbonyl, 2-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;
Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylpropyloxycarbonyl, n-Pentyloxycarbonyl, 1-Methylbutyloxycarbonyl, 2-Methylbutyloxycarbonyl, 3-Methylbutyloxycarbonyl, 1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methylpentyloxycarbonyl, 2-Methylpentenyloxycarbonyl, 3-Methylpentenyloxycarbonyl, 4-Methylpentenyloxycarbonyl, 1,2-Dimethylbutyloxycarbonyl, 1,3-Dimethylbutyloxycarbonyl, 2,3-Dimethylbutyloxycarbonyl, 1,1-Dimethylbutyloxycarbonyl, 2,2-Dimethylbutyloxycarbonyl, 3,3-Dimethylbutyloxycarbonyl, 1,1,2-Trimethylpropyloxycarbonyl, 1,2,2-Trimethylpropyloxycarbonyl, 1-Ethylbutyloxycarbonyl, 2-Ethylbutyloxycarbonyl, 1-Ethyl-2-methylpropyloxycarbonyl, n-Heptyloxycarbonyl, 1-Methylhexyloxycarbonyl, 2-Methylhexyloxycarbonyl, 3-Methylhexyloxycarbonyl, 4-Methylhexyloxycarbonyl, 5-Methylhexyloxycarbonyl, 1-Ethylpentenyloxycarbonyl, 1-Propylbutyloxycarbonyl, Octyloxycarbonyl, insbesondere Methoxycarbonyl, Ethoxycarbonyl, 1-Methylethoxycarbonyl, 1-Methylpropyloxycarbonyl;
Cycloalkyl wie Cyclopropyl, Cyclobutyl, Cyclopentyl, Cyclohexyl, Cycloheptyl;
Dialkylamino wie Dimethylamino, Diethylamino, Dipropylamino, Di-1-methylethyl, Dibutylamino, Di-1-methylpropylamino, Di-2-methylpropylamino, Di-1,1-methylethylamino, Ethylmethylamino, Propylmethylamino, 1-Methylethylmethylamino oder Butylmethylamino;
Phenyl, Phenoxy, oder Phenylcarbonyl, wobei diese aromatische Reste ihrerseits ein bis fünf Halogenatome wie vorstehend genannt, insbesondere Fluor, Chlor und Brom und/oder ein bis drei der folgenden Reste tragen können: Alkyl, Halogenalkyl, Alkoxy, und/oder Alkylthio mit jeweils ein bis vier Kohlenstoffatomen wie im allgemeinen und im besonderen vorstehend genannt;
substituiertes C₃-C₁₂-Cycloalkyl, insbesondere C₃-C₆-Cycloalkyl wie Cyclopropyl, Cyclobutyl, Cyclopentyl, Cyclohexyl; beispielsweise 1-Cyanocyclohexyl oder 1-Methyl-cyclohexyl;
Phenyl, Phenyl ein- bis dreifach substituiert durch C₁-C₄-Alkyl oder -Alkoxy wie Methyl, Ethyl, Propyl, Butyl, Methoxy, Ethoxy oder Phenyl substituiert durch ein bis fünf Halogenatome, z.B. Chlor oder Fluor;
oder R⁶ zusammen mit R⁷ eine unsubstituierte oder 1- bis 4-fach durch C₁-C₄-Alkyl, Halogen, Cyano, C₁-C₄-Alkoxy substituierte C₄-C₇-Alkylenkette wie Butylen, Pentylen, Hexylen, Heptylen, in der eine CH₂-Gruppe durch -NH, Schwefel und insbesondere Sauerstoff ersetzt sein kann, bilden. Beispielsweise seien Morpholino, Piperidino, 2,6-Dimethylmorpholino genannt;
eine Gruppe 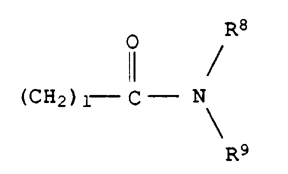 in der l für 1,2,3 oder 4 steht und R⁸ und R⁹ gleich oder verschieden sind und für Wasserstoff, C₁-C₆-Alkyl, C₃-C₆-Alkenyl oder C₃-C₆-Alkinyl jeweils wie vorstehend für R⁶, R⁷ genannt stehen;
eine Gruppe

-(CH₂)ₗ-S(=O)ₖ-R¹⁰

in der k für 0, 1 oder 2 steht, l die obengenannte Bedeutung hat und R¹⁰ abgesehen von Wasserstoff die für R⁸ bzw. R⁹ genannte Bedeutung hat;
oder R¹ ein Rest

-HN-SO₂-R¹¹,

in dem R¹¹ folgende Bedeutung hat:
C₁-C₆-Alkyl, das ein bis vier der folgenden Substituenten tragen kann: Halogen, Nitro, Cyano, C₁-C₆-Alkyl; insbesondere seien genannt: Methyl, Cyanomethyl, Ethyl, 2-Nitroethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 1-Ethyl-2-methylpropyl;
Phenyl, das ein bis vier der folgenden Substituenten tragen kann: Halogen, Nitro, Cyano, C₁-C₆-Alkyl; insbesondere seien genannt: 2-Fluorphenyl, 3-Fluorphenyl, 4-Fluorphenyl, 2,6-Difluorphenyl, 2,4-Difluorphenyl, 2,3-Difluorphenyl, 2-Fluor-4-trifluormethylphenyl, 2-Chlorphenyl, 3-Chlorphenyl, 2-Bromphenyl, 2-Iodphenyl, 2-Chlor-6-fluorphenyl, 2,4-Dichlorphenyl, 2,6-Dichlorphenyl, 3,5-Dichlorphenyl, 2-Chlor-4-fluorphenyl, 2-Chlor-6-methylphenyl, 2,3,5-Trichlorphenyl, 2-Methylphenyl, 3-Methylphenyl, 4-Methylphenyl, 2,6-Dimethylphenyl, 2,4-Dimethylphenyl, 3,5-Dimethylphenyl, 2,4,6-Trimethylphenyl, 2-Chlor-4-methylphenyl, 2-Trifluormethylphenyl, 3-Trifluormethylphenyl, 2-Cyanophenyl, 3-Cyanophenyl, 4-Cyanophenyl, 2-Nitrophenyl, 3-Nitrophenyl, 4-Nitrophenyl;
oder den Rest OR⁵, in dem
- R⁵: einen über ein Stickstoffatom gebundenen, gegebenenfalls substituierten 5-gliedrigen aromatischen Heterocyclus mit ein bis vier Stickstoffatomen darstellen, wie z.B. 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 4-Jod-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl
oder eine Gruppe 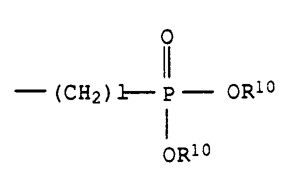 in der l und R¹⁰ die oben erwähnte Bedeutung haben;
- R²,R³: Alkyl wie z.B. Methyl, Ethyl, Propyl, 1-Methylethyl, Butyl, 1-Methylpropyl, 2-Methylpropyl, 1,1-Dimethylethyl;
Halogenalkyl wie z.B. Difluormethyl, Trifluormethyl, Chlordifluormethyl, Dichlorfluormethyl, 1-Fluorethyl, 2-Fluorethyl, 2,2-Difluorethyl, 1,1,2,2-Tetrafluorethyl, 2,2,2-Trifluorethyl, 2-Chlor-1,1,2-trifluorethyl, Pentafluorethyl, insbesondere Difluormethyl und Trifluormethyl;
Alkoxy mit ein bis vier Kohlenstoffatomen, insbesondere Alkoxy, wie z.B. Methoxy, Ethoxy, Propoxy, 1-Methylethoxy, Butoxy, 1-Methylpropoxy, 2-Methylpropoxy, 1,1-Dimethylethoxy, vorzugsweise Methoxy, Ethoxy und 1-Methylethoxy;
Halogenalkoxy wie z.B. Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy, Pentafluorethoxy, insbesondere Difluormethoxy und Trifluormethoxy;
Alkylthio mit ein bis vier Kohlenstoffatomen, insbesondere Alkylthio, wie z.B. Methylthio, Ethylthio, Propylthio, 1-Methylethylthio, Butylthio, 1-Methylpropylthio, 2-Methylpropylthio, 1,1-Dimethylethylthio, vorzugsweise Methylthio, Ethylthio und 1-Methylethylthio;
- R⁴: Wasserstoff, Cyano, Nitro, Halogen wie Fluor, Chlor oder Brom, Alkyl oder Halogenalkyl wie für R² und R³ genannt.

Im Hinblick auf die selektive herbizide Wirksamkeit sind Verbindungen I besonders bevorzugt, in denen die Substituenten folgende Bedeutung haben:
- R: Halogen, bevorzugt Chlor und Brom;
C₁-C₄-Alkyl, bevorzugt Methyl oder Ethyl;
- R¹: ein Rest ONR⁶R⁷, in dem R⁶ und R⁷
Wasserstoff, C₁-C₄-Alkyl, eine C₄-C₆-Alkylenkette oder eine C₃-C₅-Alkylenkette mit einem Heteroatom darstellen. Als besonders bevorzugt seien die Reste Wasserstoff, Methyl, Ethyl, 1,1-Dimethyl-ethyl, Butylen, Pentylen, 3-Oxapentylen genannt;
ein Rest OR⁵, in dem R⁵ 1-Pyrazolyl oder 1-Imidazolyl ist;
ein Rest NH-SO₂-R¹¹, in dem R¹¹ Methyl, Phenyl oder 4-Methylphenyl ist;
- R², R³: Methoxy;
- X: Sauerstoff oder Schwefel;
- Z: Stickstoff oder die Methingruppe;
- R⁴: Wasserstoff.

Die erfindungsgemäßen herbiziden und wachstumsregulierenden Verbindungen I bzw. die sie enthaltenden Mittel können z.B. in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier-, oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- oder Ammoniumsalze von aromatischen Sulfonsäuren, z.B. Lignin-, Phenol-, Naphthalin- und Di-butyl-naphthalinsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol- und Tributylphenolpolyglykolether, Alkylarylpolyetheralkohole, Isotridecylalkohol, Fettalkohol-Ethylenoxid-Kondensate, ethoxyliertes Ricinusöl, Polyoxyethylenalkylether oder Polyoxypropylen, Laurylalkoholpolyglykoletheracetat, Sorbitester, Lignin-sulfitablaugen oder Methylcellulose in Betracht.

Pulver-, Streu- und Stäinbemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosemehl oder andere feste Trägerstoffe.

Die Formulierungen enthalten zwischen 0.1 und 95 Gew.%, vorzugsweise zwischen 0.5 und 90 Gew.% Wirkstoff. Die Wirkstoffe werden dabei in einer Reinheit von 90% bis 100%, vorzugsweise 95% bis 100%, (nach NMR-Spektrum) eingesetzt.

Die erfindungsgemäßen Verbindungen I können beispielsweise wie folgt formuliert werden:
I. Man vermischt 90 Gewichtsteile der Verbindung Nr. 2.004 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 2.004 werden in einer Mischung gelöst, die aus 80 Gewichtsteilen Xylol, 10 Gewichtsteilen des Anlagerungsproduktes von 8 bis 10 mol Ethylenoxid an 1 mol Ölsäure-N-monoethanolamid, 5 Gewichtsteilen Calciumsalz der Dodecylbenzolsulfonsäure und 5 Gewichtsteilen des Anlagerungsproduktes von 40 mol Ethylenoxid an 1 mol Ricinusöl besteht. Durch Ausgießen und feines Verteilen der Lösung in 100000 Gewichtsteilen Wasser erhält man eine wäßrige Dispersion, die 0.02 Gew.% des Wirkstoffs enthält.
III. 20 Gewichtsteile der Verbindung 2.004 werden in einer Mischung gelöst, die aus 40 Gewichtsteilen Cyclohexanon, 30 Gewichtsteilen Isobutanol, 20 Gewichtsteilen des Anlagerungsproduktes von 7 mol Ethylenoxid an 1 mol Isooctylphenol und 10 Gewichtsteilen des Anlagerungsproduktes von 40 mol Ethylenoxid 1 mol Ricinusöl besteht. Durch Eingießen und feines Verteilen der Lösung in 100000 Gewichtsteilen Wasser erhält man eine wäßrige Dispersion, die 0.02 Gew.% des Wirkstoffs enthält.
IV. 20 Gewichtsteile des Wirkstoffs 2.004 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 mol Ethylenoxid 1 mol Ricinusöl besteht. Durch Eingießen und feines Verteilen der Lösung in 100000 Gewichtsteilen Wasser erhält man eine wäßrige Dispersion, die 0.02 Gew.% des Wirkstoffs enthält.
V. 20 Gewichtsteile des Wirkstoffs Nr. 2.004 werden mit 3 Gewichtsteilen des Natriumsalzes der Diisobutylnaphtalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0.1 Gew.% des Wirkstoffs enthält.
VI. 3 Gewichtsteile des Wirkstoffs Nr. 2.004 werden mit 97 Gewichtsteilen feinteiligem Kaolin vermischt. Man erhält auf diese Weise ein Stäubemittel, das 3 Gew.% des Wirkstoffs enthält.
VII. 30 Gewichtsteile des Wirkstoffs Nr. 2.004 werden mit einer Mischung aus 92 Gewichtsteilen pulverförmigem Kieselsäuregel 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 2.004 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit Zielpflanzen und Wachstumsstadien 0.001 bis 3.0, vorzugsweise 0.005 bis 0.5 kg/ha aktive Substanz (a.S.).

Die wachstumsregulierend wirksamen Salicylsäurederivate der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem
a) von der Pflanzenart und -sorte,
b) vom Zeitpunkt der Applikation, bezogen auf das Entwicklungsstadium der Pflanze und von der Jahreszeit,
c) von dem Applikationsort und -verfahren (z.B. Samenbeize, Bodenbehandlung, Blattapplikation oder Stamminjektion bei Bäumen),
d) von klimatischen Faktoren, z.B. Temperatur, Niederschlagsmenge, außerdem auch Tageslänge und Lichtintensität,
e) von der Bodenbeschaffenheit (einschließlich Düngung),
f) von der Formulierung bzw. Anwendungsform des Wirkstoffs und schließlich
g) von der angewendeten Konzentration der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt.
A. Mit den erfindungsgemäß verwendbaren Verbindungen läßt sich das vegetative Wachstum der Pflanzen stark hemmen, was sich insbesondere in einer Reduzierung des Längenwachstums äußert.
   Als vorteilhaft für die Praxis erweist sich eine verminderte Intensität des Wachstums von Gräsern sowie lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterinngsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide, ist es vorteilhaft, wenn die Bestände durch Behandlung mit den erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt bzw. Pflanzenmasse - dem Befall mit verschiedenen Krankheiten (z.B. Pilzkrankheit) vorgebeugt werden.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Citrusfrüchten zu erhöhen, den Proteingehalt in Getreide oder Soja zu steigern oder Gummibäume zum vermehrten Latexfluß zu stimulieren.
   Dabei können die Salicylsäurederivate der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Citrusfrüchten, Oliven oder bei anderen Arten und Sorten von Kern-, Stein- und Schalenobst ermöglicht wird. Derselbe Mechanismus, d.h. die Förderung der Ausbildung von Trenngewebe zwischen Frucht-, bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Durch den Einsatz der erfindungsgemäßen Substanzen läßt sich die Intensität der Bewässerung reduzieren und damit eine kostengünstigere Bewirtschaftung durchführen, weil u.a.
   - eine Öffnungsweite der Stomata reduziert wird,
   - eine dickere Epidermis und Cuticila ausgebildet werden,
   - die Durchwurzelung des Bodens verbessert wird und
   - das Mikroklima im Pflanzenbestand durch einen kompakteren Wuchs günstig beeinflußt wird.

Die erfindungsgemäß zu verwendenden Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0.001 bis 50 g, vorzugsweise 0.01 bis 10 g, je Kilogramm Saatgut benötigt.

Für die Blatt- und Bodenbehandlung sind im allgemeinen Gaben von 0.001 bis 10 kg/ha, bevorzugt 0.01 bis 3 kg/ha, insbesondere 0.01 bis 0.5 kg/ha als ausreichend zu betrachten.

Die Applikation der herbiziden Mittel bzw. der Wirkstoffe kann im Vorauflauf- oder Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0.001 bis 3.0, vorzugsweise 0.15 bis 1.0 kg/ha aktive Substanz (a.S.).

Besondere Bedeutung haben die Salicylsäurederivate I auch für die Bekämpfung einer Vielzahl von Pilzen an verschiedenen Kulturpflanzen wie Weizen, Roggen, Gerste, Hafter, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sich zur Bekämpfung folgender Pflanzenkrankheiten:
Erysiphe graminis (echter Mehltau) in Getreide
Erysiphe cichoracearum und Sphaerotheca fuliginea an Kürbisgewächsen,
Podosphaera leucotricha an Äpfeln,
Unicinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pilze oder die vor Pilzbefall zu schützenden Pflanzen, Saatgüter, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Der Saatgutbehandlung werden im allgemeinen Wirkstoffmengen von 0,001 bis 50 g, vorzugsweise 0,01 bis 10 g je Kilogramm Saatgut benötigt.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Zur Verbreiterung des Wirkungsspektrums und zur Erzielung synergistischer Effekte können die erfindungsgemäßen Verbindungen I mit zahlreichen Vertretern anderer herbizider und wachstumsregulierender Wirkstoffgruppen gemischt und gemeinsam ausgebracht werden. Beispielsweise kommen als Mischungspartner Diazine, (4H)-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy- und Heteroaryloxyphenoxypropionsäuren sowie deren Salze, Ester, Amide und andere in Betracht.

Außerdem kann es von Nutzen sein, die Verbindungen I allein oder in Kombination mit anderen Herbiziden auch noch mit anderen Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementemängeln eingesetzt werden. Es können auch nicht phytotoxische Öle und Ölkonzentrate zugesetzt werden.

### Synthesevorschriften:

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt. Verbindungen ohne diese Angaben lassen sich aus den entsprechenden Edukten analog synthetisieren. Die in der Tabelle wiedergegebenen Strukturen beschreiben besonders bevorzugte Wirkstoffe der Formel I.

### Beispiel 1:

### Allgemeine Vorschrift zur Herstellung aromatischer Carbonsäurehydroxylaminester oder ähnlicher Verbindungen der Formel I:

3,2 mmol der jeweiligen aromatischen 2-(4,6-Dimethoxypyrimidin-2-yl)oxycarbonsäure werden in 20 ml Dimethoxyethan vorgelegt und mit 3,2 mmol Natriumhydrid versetzt, wobei sofort eine Gasentwicklung auftritt. Es wird 1 h bei Raumtemperatur nachgerührt, auf 0°C gekühlt und mit 3,5 mmol Oxalylchlorid versetzt. Man rührt bei 0°C 1h nach und verdampft dann zur Entfernung des überschüssigen Oxalylchlorids etwa 30 % des Lösungsmittels im Vakuum. Man gibt nun 4,2 mmol des jeweiligen Hydroxylamins oder einer vergleichbaren Hydroxy-Verbindung gelöst in 10 ml Dimethoxyethan und anschließend 3,2 mmol Pyridin bei 0°C zu und erwärmt innerhalb von 1h auf Raumtemperatur. Die Mischung wird in 120 ml kaltes Wasser gegossen und mit Methylenchlorid extrahiert. Die organische Phase wird über Natriumsulfat getrocknet und im Vakuum eingeengt. Die zurückbleibende Substanz kann durch Chromatographie an Silica-Gel weiter gereinigt werden.

### Beispiel 2:

### Allgemeine Vorschrift zur Herstellung aromatischer Carbonsäurehydroxylaminester oder ähnlicher Verbindungen der Formel I:

10 mmol der entsprechenden 6-Aryl-2-(4,6-dimethoxypyrimidin-2-yloxy)-benzoesäure werden in 30 ml Tetrahydrofuran gelöst und mit 1,75 g (10,8 mmol) N,N'-Carbonylbisimidazol versetzt. Nach 30 min Rühren bei Raumtemperatur fügt man 9,9 mmol der entsprechenden Hydroxyverbindung hinzu und rührt weitere 14 h. Der Ansatz wird sodann mit 300 ml 1N-Phosphorsäure hydrolysiert und das resultierende Gemisch mehrfach mit Methyl-tert.-butylether extrahiert. Die organischen Phasen werden über Natriumsulfat getrocknet und im Vakuum eingeengt. Der verbleibende Rückstand wird durch Säulenchromatographie oder Umkristallisieren weiter gereinigt.

### Beispiel 3:

### Allgemeine Vorschrift zur Herstellung aromatischer Carbonsäurehydroxylaminester oder ähnlicher Verbindungen der Formel I:

10 mmol der entsprechenden 6-Aryl-salicylsäure werden in 30 ml Dioxan gelöst und mit 1,75 g (10,8 mmol) N,N'-Carbonylbisimidazol versetzt. Nach 30 min Rühren bei Raumtemperatur fügt man 9,9 mmol der entsprechenden Hydroxyverbindung hinzu und rührt weitere 14 h. Das Reaktionsgemisch wird sodann mit 300 ml 1N-Phosphorsäure hydrolysiert und anschließend mehrfach mit Methyl-tert.-butylether extrahiert. Die organischen Phasen werden vereint, über Natriumsulfat getrocknet und im Vakuum eingeengt. Der Rückstand wird mit 40 ml Dimethylformamid aufgenommen und mit 280 mg Natriumhydrid (85 % in Paraffin, 10 mmol) versetzt. Nach 30 min Rühren bei Raumtemperatur fügt man 1,97 g (9 mmol) 4,6-Dimethoxy-2-methylsulfonyl-pyrimidin hinzu und rührt 14 h nach. Das Gemisch wird auf 300 ml 0.1 n Phosphorsäure gegeben und mit Diethylether extrahiert. Die Etherphase wird über Natriumsulfat getrocknet, eingeengt und der Rückstand durch Säulenchromatographie oder Umkristallisieren gereinigt.

### Beispiel 4:

Zu 15 mmol 6-Aryl-salicylsäureazolylester, gelöst in 25 ml getrocknetem Dimethylformamid werden bei 10°C 0,46 g (0,015 mol) Natriumhydrid (80 %ig) gegeben und bei 30°C 3 h nachgerührt. Dann werden 3,27 g (0,015 mol) 2-Methylsulfonyl-4,6-dimethoxypyrimidin zugegeben und 12 h bei Raumtemperatur nachgerührt. Das Reaktionsgemisch wird in 500 ml Wasser gegeben, dem vorher 2,5 ml Orthophosphorsäure zugesetzt wurden. Das sich abscheidende Öl wird in Essigsäureethylester aufgenommen und über Natriumsulfat getrocknet. Der nach dem Einengen verbleibende Rückstand wird durch Säulenchromatographie oder Umkristallisieren gereinigt.

### Beispiel 5:

### 6-(4-Bromphenyl)-2-(4,6-dimethoxypyrimidin-2-yloxy)-1-(N,N-dimethylaminooxy-carbonyl)-benzol (Beispiel 2.004)

3.35 g (10 mmol) 3-(4-Bromphenyl)-2-(N,N-dimethylaminooxycarbonyl)phenol werden in 25 ml getrocknetem Dimethylformamid gelöst, bei 10°C mit 0,30 g (0,01 mol) Natriumhydrid (80 %ig) versetzt und bei 30°C 3h nachgerührt. Dann werden 2,18 g (0,01 mol) 2-Methylsulfonyl-4,6-dimethoxypyrimidin zugegeben und 12 h bei Raumtemperatur nachgerührt. Nach Aufarbeitung gemäß Beispiel 4 erhält man einen farblosen Feststoff.

### Beispiel 6:

### 6-(4-Chlorphenyl)-2-(4,6-dimethoxypyrimidin-2-yloxy)-1-(N,N-dimethylaminooxy-carbonyl)-benzol (Beispiel 2.003)

1.24 g (3,2 mmol) 6-(4-Chlorphenyl)-2-(4,6-dimethoxypyrimidin-2-yl)benzoesäure werden in 20 ml Dimethoxyethan vorgelegt und mit 3,2 mmol Natriumhydrid versetzt, wobei sofort eine Gasentwicklung auftritt. Es wird 1 h bei Raumtemperatur nachgerührt, auf 0°C gekühlt und mit 3,5 mmol Oxalylchlorid versetzt. Man rührt bei 0°C 1 h nach und verdampft dann zur Entfernung des überschüssigen Oxalylchlorids etwa 30 % des Lösungsmittels im Vakuum. Man gibt nun eine Lösung von 4,2 mmol N-N-Dimethylhydroxylamin in 10 ml Dimethoxyethan (aus 230 mg N,N-Dimethylhydroxylamin-hydrochlorid und 332 mg Pyridin) und anschließend 3,2 mmol Pyridin bei 0°C zu und erwärmt innerhalb von 1 h auf Raumtemperatur. Die Mischung wird in 120 ml kaltes Wasser gegossen und mit Methylenchlorid extrahiert. Die organische Phase wird über Natriumsulfat getrocknet und im Vakuum eingeengt. Das zurückbleibende Öl wird durch Chromatogrophie an Silicagel weiter gereinigt.

### Beispiel 7:

### 6-(2-Methylphenyl)-2-(3,5-dimethoxy-s-triazin-1-yloxy)-1-[(1-pyrazolyl)oxy-carbonyl]-benzol (Beispiel 1.022)

2.12 g (10 mmol) 6-(2-Methylphenyl)salicylsäure werden in 30 ml Tetrahydrofuran gelöst und mit 1.75 g (10,8 mmol) N,N'-Carbonylbisimidazol versetzt. Nach 30 min Rühren bei Raumtemperatur fügt man 9,9 mmol N-Hydroxypyrazol hinzu und rührt weitere 14 h. Das Reaktionsgemisch wird sodann mit 300 ml 1N-Phosphorsäure hydrolysiert und mehrfach mit Methyl-tert.-butylether extrahiert. Die organischen Phasen werden vereint, über Natriumsulfat getrocknet und im Vakuum eingeengt. Der Rückstand wird mit 40 ml Dimethylformamid aufgenommen und mit 280 mg Natriumhydrid (85 % in Paraffin, 10 mmol) versetzt. Nach 30 min Rühren bei Raumtemperatur fügt man 1,58 g (9 mmol) 1-Chlor-3,5-dimethoxy-s-triazin hinzu und rührt 14 h nach. Das Gemisch wird auf 300 ml 0.1 n Phosphorsäure gegeben und mit Diethylether extrahiert. Die Etherphase wird über Natriumsulfat getrocknet, eingeengt und der Rückstand durch Säulenchromatographie gereinigt.

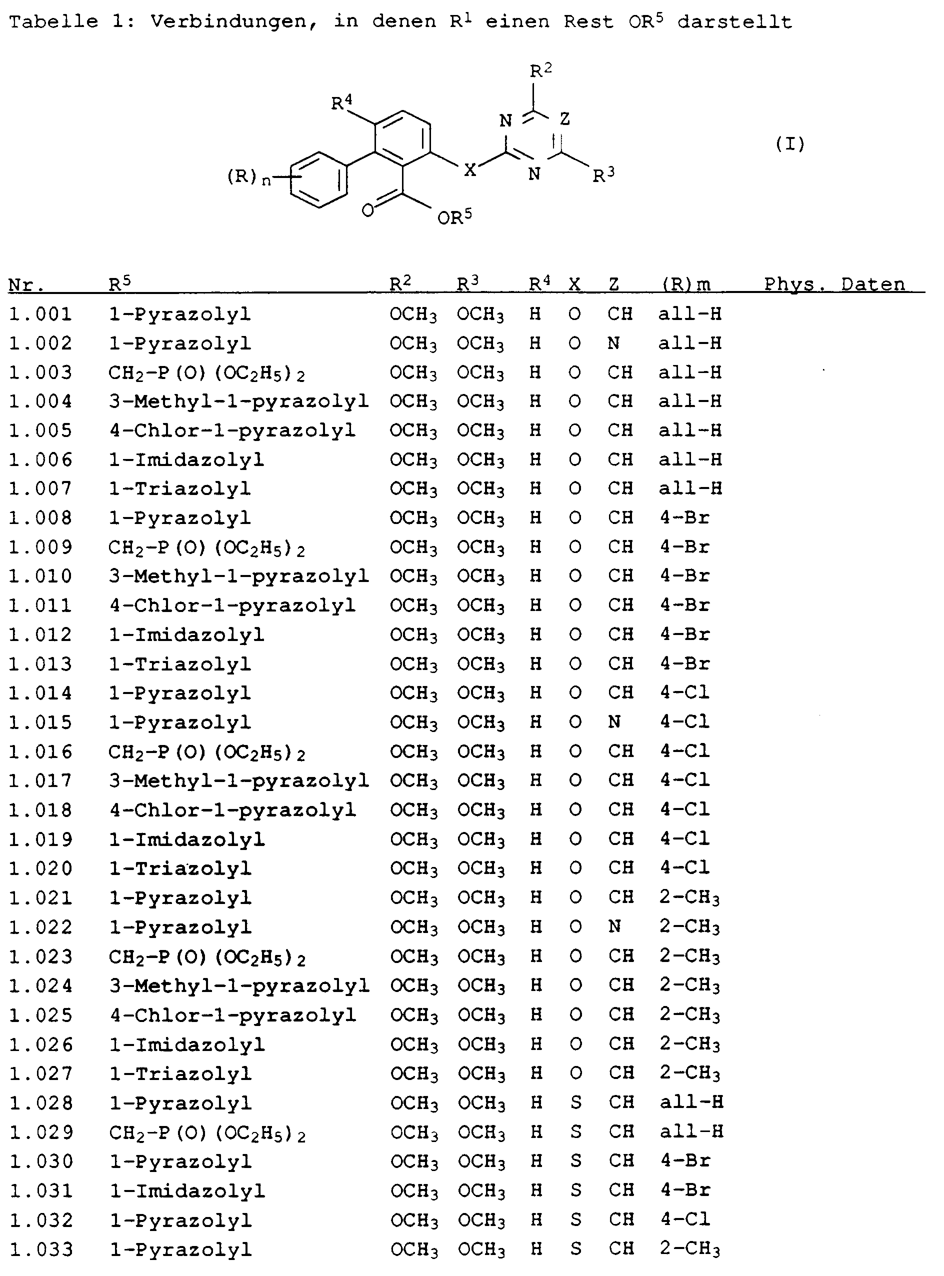

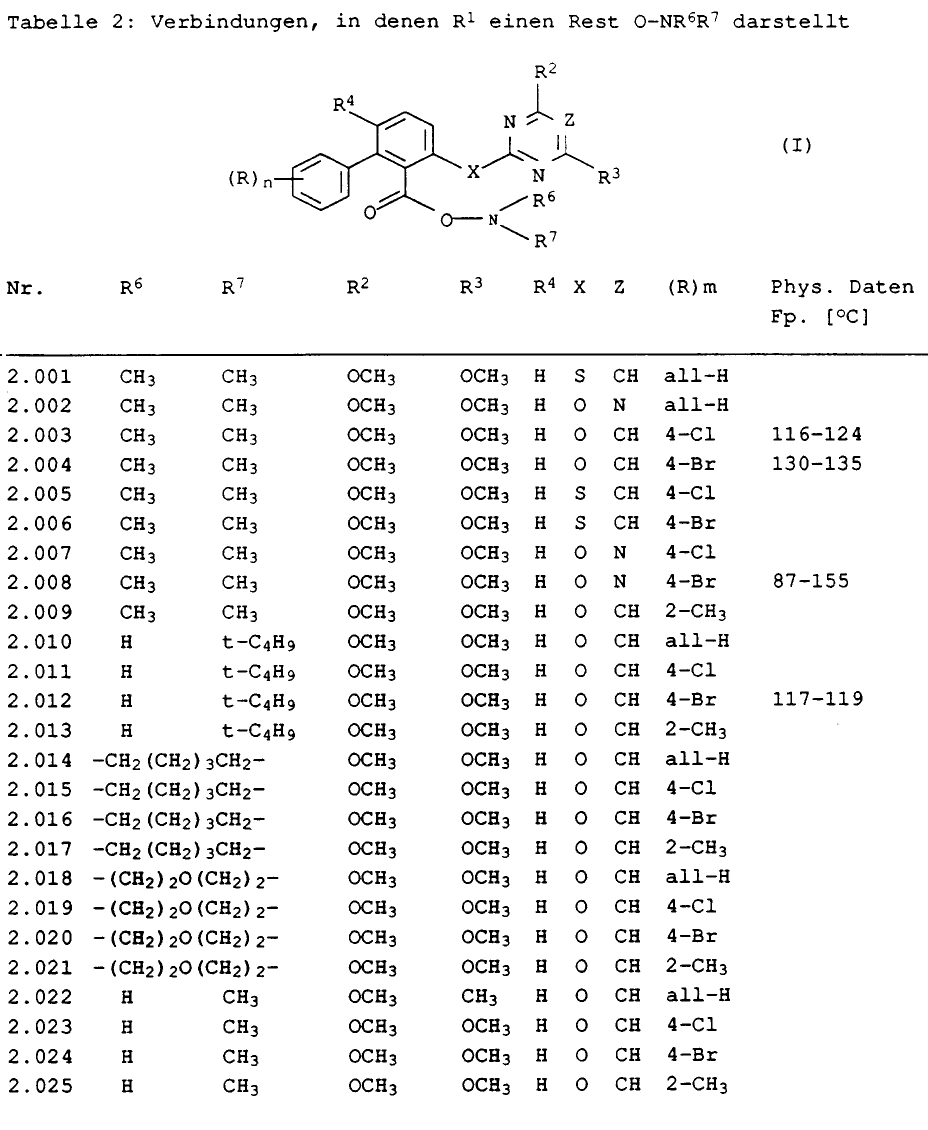

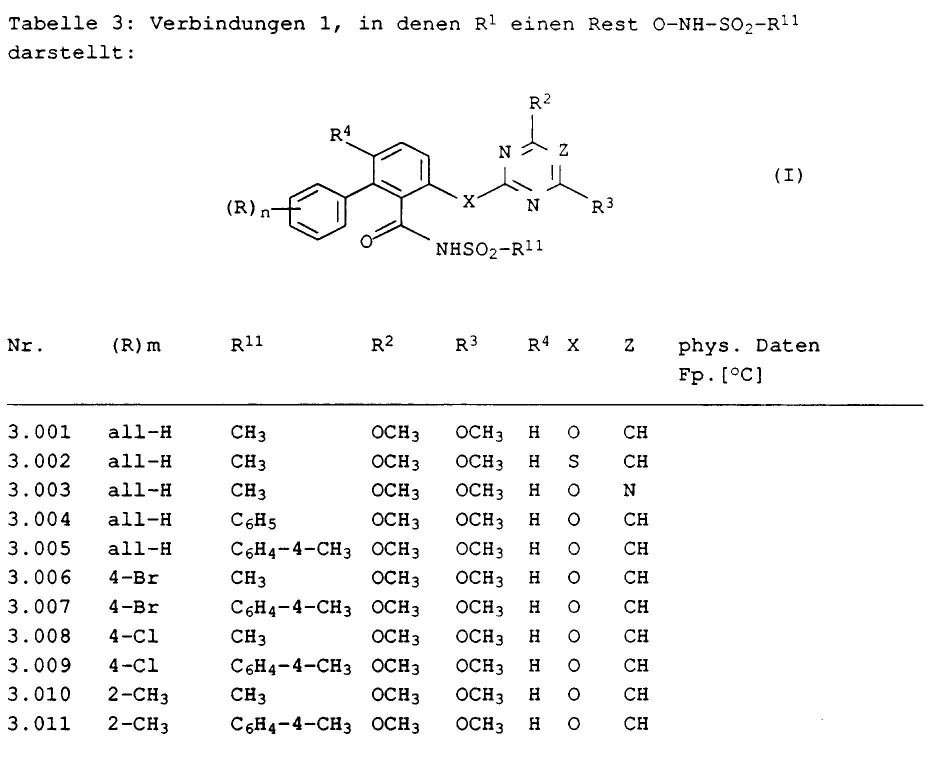

### Anwendungsbeispiele

Die herbizide Wirkung der Salicylsäurederivate der Formel I ließ sich durch Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, Um Keimung und Wachstum zu fördern, und anschließend mit durchsichtigen Plastikhauben abgedeckt, bis die Pflanzen angewachsen waren. Diese Abdeckung bewirkt ein gleichmaßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,03 bzw. 0,015 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde aufgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name | Englischer Name |
|---|---|---|---|
| HORVS | Hordeum vulgaris | Sommergerste | spring barley |
| TRZAS | Triticum aestivum | Sommerweizen | spring wheat |
| ALOMY | Alopecurus myosuroides | Ackerfuchsschwanz | blackgrass |
| STEME | Stellaria media | Vogelsternmiere | chickweed |
| SOLNI | Solanum nigrum | Schwarzer Nachschatten | black nightshade |

Der nachfolgende Vergleichsversuch zwischen Verbindung 2.004 und Verbindungen Nr. 44 aus EP-A 426 476 (Vergleichssubstanz A) demonstriert die überraschend hohe Selektivität der erfindungsgemäßen Verbindungen in den Beispielkulturen Sommergerste und Sommerweizen bei sehr guter herbizider Aktivität. Aufgrund der untragbar hohen Schädigungen ist die Vergleichssubstanz A in diesen Kulturen nicht zu verwenden.

Die Versuchsergebnisse sind in Tabelle I zusammengestellt.

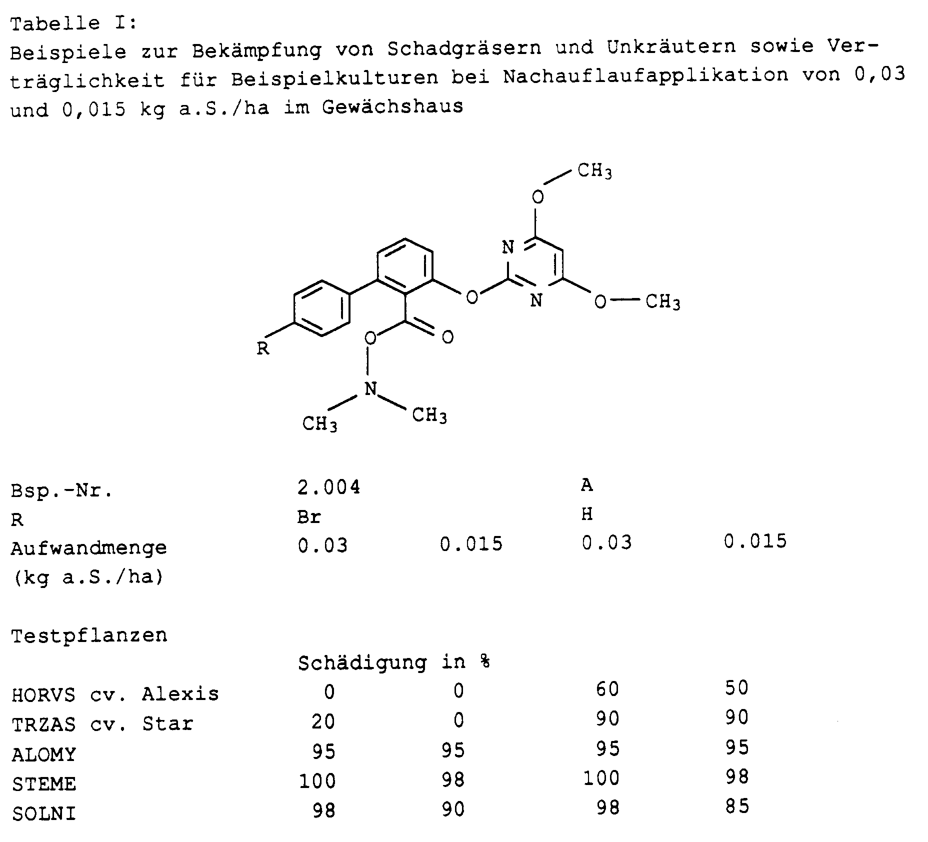

## Patentansprüche (Patentansprüche für folgende(n) Vertragsstaat(en): AT, BE, CH, DE, FR, GB, IT, LI, NL, SE)

1. Salicylsäurederivate und deren Schwefelanaloga der Formel I, 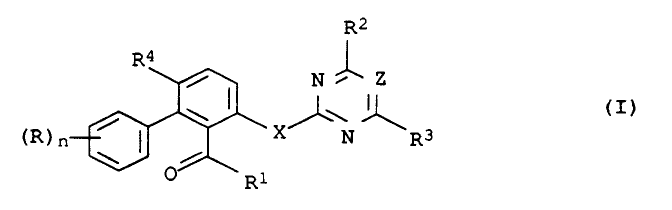 in der die Substituenten folgende Bedeutung haben:
R Halogen, C₁-C₄-Alkyl, C₁-C₄-Halogenalkyl, C₁-C₄-Alkoxy, C₁-C₄-Halogenalkoxy und/oder C₁-C₄-Alkylthio;
n eine Zahl von 1 bis 3, im Falle von Halogen als Substituenten eine Zahl von 1 bis 5
R¹ ein Rest 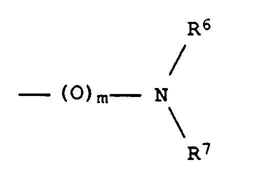 in dem m für 0 und 1 steht und R⁶ und R⁷ die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, bis-C₁-C₆-Dialkylamino, C₃-C₆-Cycloalkyl, gegebenenfalls substituiertes Phenyl;
gegebenenfalls substituiertes C₃-C₆-Cycloalkyl;
gegebenenfalls substituiertes Phenyl;
oder R⁶ zusammen mit R⁷ eine gegebenenfalls substituierte C₄-C₇-Alkylenkette bildet, in der eine CH₂-Gruppe durch Sauerstoff, Schwefel oder -NH ersetzt sein kann;
oder R¹ eine Gruppe 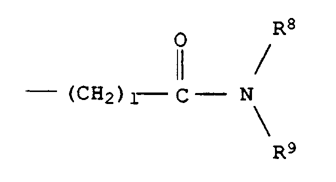 in der R⁸ und R⁹, für Wasserstoff, C₁-C₆-Alkyl, C₃-C₆-Alkenyl oder C₃-C₆-Alkinyl stehen und l die Werte 1, 2, 3 oder 4 annimmt;
oder R¹ eine Gruppe
-(CH₂)ₗ-S(=O)ₖ-R¹⁰
in der R¹⁰ für C₁-C₆-Alkyl, C₃-C₆-Alkenyl oder C₃-C₆-Alkinyl, l für 1, 2, 3 oder 4 und k für 0, 1 oder 2 stehen;
oder R¹ ein Rest
-HN-SO₂-R¹¹,
in dem R¹¹ für die Reste C₁-C₆-Alkyl oder Phenyl steht, die ihrerseits ein bis vier der folgenden Substituenten tragen können: Halogen, Nitro, Cyano, C₁-C₆-Alkyl;
oder R¹ ein Rest OR⁵, worin
R⁵ einen über ein Stickstoffatom gebundenen gegebenenfalls substituierten 5-gliedrigen aromatischen Heterocyclus mit ein bis vier Stickstoffatomen im Ring;
oder R¹ einen Rest 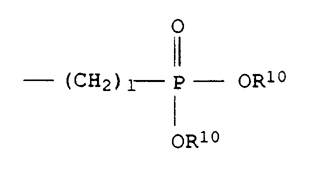 in dem R¹⁰ und l die oben genannte Bedeutung haben, darstellt;
wobei der Ausdruck gegebenenfalls substituiert in den voranstehenden Definitionen bedeutet, daß die so bezeichneten Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio;
R², R³ C₁-C₄-Alkyl, C₁-C₄-Halogenalkyl, C₁-C₄-Alkoxy, C₁-C₄-Halogenalkoxy und/oder C₁-C₄-Alkylthio;
R⁴ Wasserstoff, Halogen, Nitro, C₁-C₄-Alkyl, Cyano oder C₁-C₄-Halogenalkyl;
X ein Sauerstoff- oder Schwefelatom;
Z Stickstoff oder die Methingruppe.

2. Salicylsäurederivate der Formel I gemäß Anspruch 1, in der n für 1, 2 oder 3 und die Substituenten die folgende Bedeutung haben:
R Halogen, C₁-C₄-Alkyl;
R¹ ein Rest ONR⁶R⁷, in dem R⁶ und R⁷ Wasserstoff oder C₁-C₄-Alkyl bedeuten oder R⁶ mit R⁷ eine C₄-C₆-Alkylenkette bildet, in der eine CH₂-Gruppe durch Sauerstoff oder Schwefel ersetzt sein kann;
ein Rest OR⁵, in dem R⁵ 1-Pyrazolyl oder 1-Imidazolyl ist;
NHSO₂CH₃, NHSO₂C₆H₅;
R², R³ Methoxy;
R⁴ Wasserstoff;
X Sauerstoff oder Schwefel;
Z Stickstoff oder die Methingruppe.

3. Salicylsäurederivate der Formel I gemäß Anspruch 1, in der R¹ O-N(CH₃)₂, X Sauerstoff, Y Stickstoff, Z die Methingruppe, R² und R³ Methoxy darstellen und (R) 4-Cl, 4-Br oder 2-CH₃ ist.

4. Herbizides Mittel, enthaltend eine Verbindung der Formel I gemäß Anspruch 1 und übliche inerte Zusatzstoffe.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge einer Verbindung I gemäß Anspruch 1 behandelt.

6. Verwendung von Salicylsäurederivaten und deren Schwefelanalogen der Formel I gemäß Anspruch 1 als Herbizide.

7. Mittel zur Beeinflussung des Pflanzenwachstums, enthaltend ein Salicylsäurederivat der Formel I gemaß Anspruch 1 und übliche inerte Zusatzstoffe.

8. Verfahren zur Regulierung des Pflanzenwachstums, dadurch gekennzeichnet, daß man eine regulativ wirksame Menge eines Salicylsäurederivats der allgemeinen Formel I gemäß Anspruch 1 auf die Samen, die Pflanzen und/oder deren Lebensraum einwirken läßt.

9. Fungizides und/oder nitrifikationshemmendes Mittel, enthaltend ein Salicylsäurederivat der Formel I gemäß Anspruch 1 und übliche inerte Zusatzstoffe.

## Patentansprüche (Patentansprüche für folgende(n) Vertragsstaat(en): ES)

1. Herbizides Mittel, enthaltend Salicylsäurederivate und deren Schwefelanaloga der Formel I, 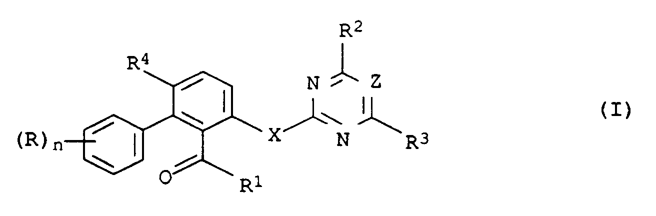 in der die Substituenten folgende Bedeutung haben:
R Halogen, C₁-C₄-Alkyl, C₁-C₄-Halogenalkyl, C₁-C₄-Alkoxy, C₁-C₄-Halogenalkoxy und/oder C₁-C₄-Alkylthio;
n eine Zahl von 1 bis 3, im Falle von Halogen als Substituenten eine Zahl von 1 bis 5
R¹ ein Rest 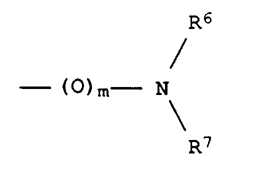 in dem m für 0 und 1 steht und R⁶ und R⁷ die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, bis-C₁-C₆-Dialkylamino_{,} C₃-C₆-Cycloalkyl, gegebenenfalls substituiertes Phenyl;
gegebenenfalls substituiertes C₃-C₆-Cycloalkyl;
gegebenenfalls substituiertes Phenyl;
oder R⁶ zusammen mit R⁷ eine gegebenenfalls substituierte C₄-C₇-Alkylenkette bildet, in der eine CH₂-Gruppe durch Sauerstoff, Schwefel oder -NH ersetzt sein kann;
oder R¹ eine Gruppe 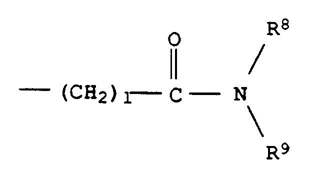 in der R⁸ und R⁹, für Wasserstoff, C₁-C₆-Alkyl, C₃-C₆-Alkenyl oder C₃-C₆-Alkinyl stehen und l die Werte 1, 2, 3 oder 4 annimmt;
oder R¹ eine Gruppe
-(CH₂)ₗ-S(=O)ₖ-R¹⁰
in der R¹⁰ für C₁-C₆-Alkyl, C₃-C₆-Alkenyl oder C₃-C₆-Alkinyl, l für 1, 2, 3 oder 4 und k für 0, 1 oder 2 stehen;
oder R¹ ein Rest
-HN-SO₂-R¹¹,
in dem R¹¹ für die Reste C₁-C₆-Alkyl oder Phenyl steht, die ihrerseits ein bis vier der folgenden Substituenten tragen können: Halogen, Nitro, Cyano, C₁-C₆-Alkyl;
oder R¹ ein Rest OR⁵, worin
R⁵ einen über ein Stickstoffatom gebundenen gegebenenfalls sübstituierten 5-gliedrigen aromatischen Heterocyclus mit ein bis vier Stickstoffatomen im Ring;
oder R¹ einen Rest 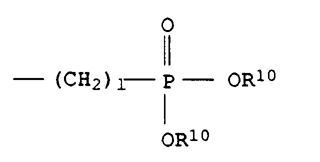 in dem R¹⁰ und l die oben genannte Bedeutung haben, darstellt;
wobei der Ausdruck gegebenenfalls substituiert in den voranstehenden Definitionen bedeutet, daß die so bezeichneten Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio;
R², R³ C₁-C₄-Alkyl, C₁-C₄-Halogenalkyl, C₁-C₄-Alkoxy, C₁-C₄-Halogenalkoxy und/oder C₁-C₄-Alkylthio;
R⁴ Wasserstoff, Halogen, Nitro, C₁-C₄-Alkyl, Cyano oder C₁-C₄-Halogenalkyl;
X ein Sauerstoff- oder Schwefelatom;
Z Stickstoff oder die Methingruppe.

2. Mittel, enthaltend Salicylsäurederivate der Formel I gemäß Anspruch 1, in der n für 1, 2 oder 3 und die Substituenten die folgende Bedeutung haben:
R Halogen, C₁-C₄-Alkyl;
R¹ ein Rest ONR⁶R⁷, in dem R⁶ und R⁷ Wasserstoff oder C₁-C₄-Alkyl bedeuten oder R⁶ mit R⁷ eine C₄-C₆-Alkylenkette bildet, in der eine CH₂-Gruppe durch Sauerstoff oder Schwefel ersetzt sein kann;
ein Rest OR⁵, in dem R⁵ 1-Pyrazolyl oder 1-Imidazolyl ist;
NHSO₂CH₃, NHSO₂C₆H₅;
R², R³ Methoxy;
R⁴ Wasserstoff;
X Sauerstoff oder Schwefel;
Z Stickstoff oder die Methingruppe.

3. Mittel enthaltend, Salicylsäurederivate der Formel I gemäß Anspruch 1, in der R¹ O-N(CH₃)₂, X Sauerstoff, Y Stickstoff, Z die Methingruppe, R² und R³ Methoxy darstellen und (R) 4-Cl, 4-Br oder 2-CH₃ ist.

4. Herbizides Mittel, enthaltend eine Verbindung der Formel I gemäß Anspruch 1 und übliche inerte Zusatzstoffe.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge einer Verbindung I gemäß Anspruch 1 behandelt.

6. Verwendung von Salicylsäurederivaten und deren Schwefelanalogen der Formel I gemäß Anspruch 1 als Herbizide.

7. Mittel zur Beeinflussung des Pflanzenwachstums, enthaltend ein Salicylsäurederivat der Formel I gemaß Anspruch 1 und übliche inerte Zusatzstoffe.

8. Verfahren zur Regulierung des Pflanzenwachstums, dadurch gekennzeichnet, daß man eine regulativ wirksame Menge eines Salicylsäurederivats der allgemeinen Formel I gemäß Anspruch 1 auf die Samen, die Pflanzen und/oder deren Lebensraum einwirken läßt.

9. Fungizides und/oder nitrifikationshemmendes Mittel, enthaltend ein Salicylsäurederivat der Formel I gemäß Anspruch 1 und übliche inerte Zusatzstoffe.

## Claims (Claims for the following Contracting State(s): AT, BE, CH, DE, FR, GB, IT, LI, NL, SE)

1. A salicylic acid derivative or a sulfur analog thereof of the formula I 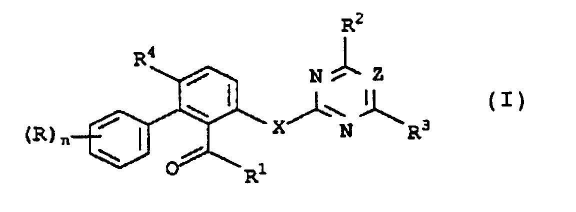 where
R is halogen, C₁-C₄-alkyl, C₁-C₄-haloalkyl, C₁-C₄-alkoxy, C₁-C₄-haloalkoxy or C₁-C₄-alkylthio;
n is 1, 2 or 3 or, in the case of halogen as substituent, 1, 2, 3, 4 or 5,
R¹ is a radical 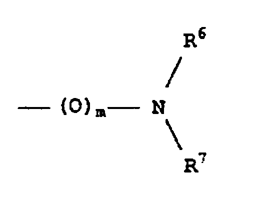 where m is 0 or 1 and R⁶ and R⁷ have the following meanings:
hydrogen;
C₁-C₆-alkyl, C₃-C₆-alkenyl or C₃-C₆-alkynyl, where these radicals may each carry from one to five halogen atoms and/or one or two of the following groups: C₁-C₆-alkoxy, C₃-C₆-alkenyloxy, C₃-C₆-alkynyloxy, C₁-C₆-alkylthio, C₃-C₆-alkenylthio, C₃-C₆-alkynylthio, C₁-C₆-haloalkoxy, cyano, C₁-C₆-alkylcarbonyl, C₃-C₆-alkenylcarbonyl, C₃-C₆-alkynylcarbonyl, C₁-C₆-alkoxycarbonyl, C₃-C₆-alkenyloxycarbonyl, C₃-C₆-alkynyloxycarbonyl, bis-C₁-C₆-dialkylamino, C₃-C₆-cycloalkyl or unsubstituted or substituted phenyl;
unsubstituted or substituted C₃-C₆-cycloalkyl;
unsubstituted or substituted phenyl;
or R⁶ together with R⁷ forms an unsubstituted or substituted C₄-C₇-alkylene chain in which a CH₂ group may be replaced by oxygen, sulfur or -NH;
or R¹ is a group 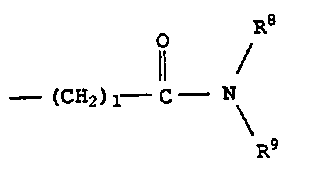 where R⁸ and R⁹ are each hydrogen, C₁-C₆-alkyl, C₃-C₆-alkenyl or C₃-C₆-alkynyl and l is 1, 2, 3 or 4;
or R¹ is a group
-(CH₂)ₗ-S(=O)ₖ-R¹⁰
where R¹⁰ is C₁-C₆-alkyl, C₃-C₆-alkenyl or C₃-C₆-alkynyl, l is 1, 2, 3 or 4 and k is 0, 1 or 2;
or R¹ is a radical
-HN-SO₂-R¹¹
where R¹¹ is C₁-C₆-alkyl or phenyl which in turn may carry from one to four of the following substituents: halogen, nitro, cyano or C₁-C₆-alkyl;
or R¹ is a radical OR⁵, where
R⁵ is an unsubstituted or substituted 5-membered aromatic heterocyclic structure which is bonded via a nitrogen atom and has from one to four nitrogen atoms in the ring;
or R¹ is a radical 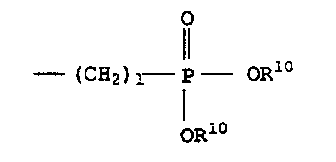 where R¹⁰ and l have the abovementioned meanings,
the expression unsubstituted or substituted in the above definitions meaning that the groups designated in this manner may carry one or more of the following substituents: halogen, nitro, cyano, C₁-C₆-alkyl, C₁-C₆-haloalkyl, C₁-C₆-alkoxy or C₁-C₆-alkylthio;
R² and R³ are each C₁-C₄-alkyl, C₁-C₄-haloalkyl, C₁-C₄-alkoxy, C₁-C₄-haloalkoxy and/or C₁-C₄-alkylthio;
R⁴ is hydrogen, halogen, nitro, C₁-C₄-alkyl, cyano or C₁-C₄-haloalkyl;
X is oxygen or sulfur, and
Z is nitrogen or the methine group.

2. A salicylic acid derivative of the formula I as claimed in claim 1, where n is 1, 2 or 3 and the substituents have the following meanings:
R is halogen or C₁-C₄-alkyl;
R¹ is a radical ONR⁶R⁷, where R⁶ and R⁷ are each hydrogen or C₁-C₄-alkyl or R⁶ together with R⁷ forms a C₄-C₆-alkylene chain in which a CH₂ group may be replaced with oxygen or sulfur;
a radical OR⁵, where R⁵ is 1-pyrazolyl or 1-imidazolyl;
NHSO₂CH₃ or NHSO₂C₆H₅;
R² and R³ are each methoxy;
R⁴ is hydrogen;
X is oxygen or sulfur; and
Z is nitrogen or the methine group.

3. A salicylic acid derivative of the formula I as claimed in claim 1, where R¹ is O-N(CH₃)₂, X is oxygen, Y is nitrogen, Z is the methine group, R² and R³ are each methoxy and (R) is 4-Cl, 4-Br or 2-CH₃.

4. A herbicide containing a compound of the formula I as claimed in claim 1 and conventional inert additives.

5. A method for controlling undesirable plant growth, wherein the undesirable plants and/or their habitat are treated with a herbicidal amount of a compound I as claimed in claim 1.

6. Use of a salicylic acid derivative or a sulfur analog thereof of the formula I as claimed in claim 1 as a herbicide.

7. An agent for influencing plant growth, containing a salicylic acid derivative of the formula I as claimed in claim 1 and conventional inert additives.

8. A method for regulating plant growth, wherein an amount, having a regulatory action, of a salicylic acid derivative of the formula I as claimed in claim 1 is allowed to act on the seeds, the plants and/or their habitat.

9. A fungicide or nitrification inhibitor containing a salicylic acid derivative of the formula I as claimed in claim 1 and conventional inert additives.

## Claims (Claims for the following Contracting State(s): ES)

1. A herbicide containing a salicylic acid derivative or a sulfur analog thereof of the formula I 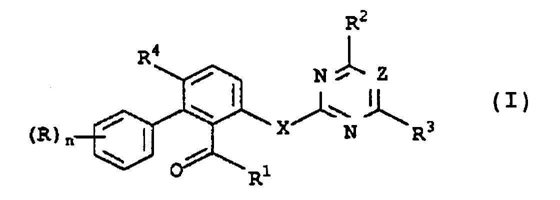 where
R is halogen, C₁-C₄-alkyl, C₁-C₄-haloalkyl, C₁-C₄-alkoxy, C₁-C₄-haloalkoxy or C₁-C₄-alkylthio;
n is 1, 2 or 3 or, in the case of halogen as substituent, 1, 2, 3, 4 or 5,
R¹ is a radical 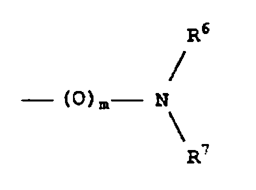 where m is 0 or 1 and R⁶ and R⁷ have the following meanings:
hydrogen;
C₁-C₆-alkyl, C₃-C₆-alkenyl or C₃-C₆-alkynyl, where these radicals may each carry from one to five halogen atoms and/or one or two of the following groups: C₁-C₆-alkoxy, C₃-C₆-alkenyloxy, C₃-C₆-alkynyloxy, C₁-C₆-alkylthio, C₃-C₆-alkenylthio, C₃-C₆-alkynylthio, C₁-C₆-haloalkoxy, cyano, C₁-C₆-alkylcarbonyl, C₃-C₆-alkenylcarbonyl, C₃-C₆-alkynylcarbonyl, C₁-C₆-alkoxycarbonyl, C₃-C₆-alkenyloxycarbonyl, C₃-C₆-alkynyloxycarbonyl, bis-C₁-C₆-dialkylamino, C₃-C₆-cycloalkyl or unsubstituted or substituted phenyl;
unsubstituted or substituted C₃-C₆-cycloalkyl;
unsubstituted or substituted phenyl;
or R⁶ together with R⁷ forms an unsubstituted or substituted C₄-C₇-alkylene chain in which a CH₂ group may be replaced by oxygen, sulfur or -NH;
or R¹ is a group 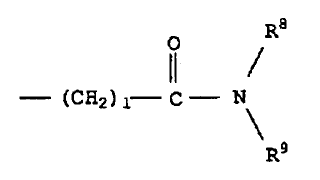 where R⁸ and R⁹ are each hydrogen, C₁-C₆-alkyl, C₃-C₆-alkenyl or C₃-C₆-alkynyl and l is 1, 2, 3 or 4;
or R¹ is a group
-(CH₂)ₗ-S(=O)ₖ-R¹⁰
where R¹⁰ is C₁-C₆-alkyl, C₃-C₆-alkenyl or C₃-C₆-alkynyl, l is 1, 2, 3 or 4 and k is 0, 1 or 2;
or R¹ is a radical
-HN-SO₂-R¹¹
where R¹¹ is C₁-C₆-alkyl or phenyl which in turn may carry from one to four of the following substituents: halogen, nitro, cyano or C₁-C₆-alkyl;
or R¹ is a radical OR⁵, where
R⁵ is an unsubstituted or substituted 5-membered aromatic heterocyclic structure which is bonded via a nitrogen atom and has from one to four nitrogen atoms in the ring;
or R¹ is a radical 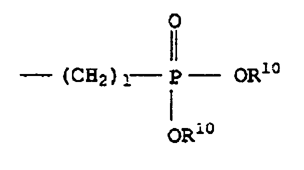 where R¹⁰ and l have the abovementioned meanings,
the expression unsubstituted or substituted in the above definitions meaning that the groups designated in this manner may carry one or more of the following substituents: halogen, nitro, cyano, C₁-C₆-alkyl, C₁-C₆-haloalkyl, C₁-C₆-alkoxy or C₁-C₆-alkylthio;
R² and R³ are each C₁-C₄-alkyl, C₁-C₄-haloalkyl, C₁-C₄-alkoxy, C₁-C₄-haloalkoxy or C₁-C₄-alkylthio;
R⁴ is hydrogen, halogen, nitro, C₁-C₄-alkyl, cyano or C₁-C₄-haloalkyl;
X is oxygen or sulfur, and
Z is nitrogen or the methine group.

2. An agent containing a salicylic acid derivative of the formula I as claimed in claim 1, where n is 1, 2 or 3 and the substituents have the following meanings:
R is halogen or C₁-C₄-alkyl;
R¹ is a radical ONR⁶R⁷, where R⁶ and R⁷ are each hydrogen or C₁-C₄-alkyl or R⁶ together with R⁷ forms a C₄-C₆-alkylene chain in which a CH₂ group may be replaced with oxygen or sulfur;
a radical OR⁵, where R⁵ is 1-pyrazolyl or 1-imidazolyl;
NHSO₂CH₃ or NHSO₂C₆H₅;
R² and R³ are each methoxy;
R⁴ is hydrogen;
X is oxygen or sulfur; and
Z is nitrogen or the methine group.

3. An agent containing a salicylic acid derivative of the formula I as claimed in claim 1, where R¹ is O-N(CH₃)₂, X is oxygen, Y is nitrogen, Z is the methine group, R² and R³ are each methoxy and (R) is 4-Cl, 4-Br or 2-CH₃.

4. A herbicide containing a compound of the formula I as claimed in claim 1 and conventional inert additives.

5. A method for controlling undesirable plant growth, wherein the undesirable plants and/or their habitat are treated with a herbicidal amount of a compound I as claimed in claim 1.

6. Use of a salicylic acid derivative or a sulfur analog thereof of the formula I as claimed in claim 1 as a herbicide.

7. An agent for influencing plant growth, containing a salicylic acid derivative of the formula I as claimed in claim 1 and conventional inert additives.

8. A method for regulating plant growth, wherein an amount, having a regulatory action, of a salicylic acid derivative of the formula I as claimed in claim 1 is allowed to act on the seeds, the plants and/or their habitat.

9. A fungicide or nitrification inhibitor containing a salicylic acid derivative of the formula I as claimed in claim 1 and conventional inert additives.

## Revendications (Revendications pour l'(les) Etat(s) contractant(s) suivant(s): AT, BE, CH, DE, FR, GB, IT, LI, NL, SE)

1. Dérivés d'acide salicylique et leurs analogues soufrés de formule I, 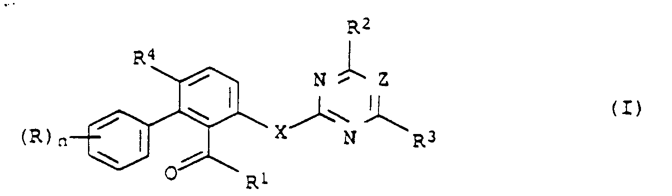 où les substituants désignent les atomes ou groupes suivants:
R halogène, alkyle en C₁-C₄, halogénoalkyle en C₁-C₄, alcoxy en C₁-C₄, halogénoalcoxy en C₁-C₄ et/ou alkylthio en C₁-C₄;
n un nombre de 1 à 3 et, dans le cas d'un halogène comme substituant, un nombre de 1 à 5,
R¹ un reste 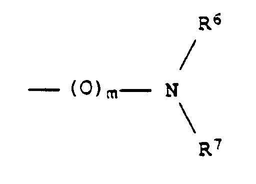 dans lequel m vaut 0 ou 1 et où R⁶ et R⁷ désignent les atomes ou groupes suivants:
hydrogène,
alkyle en C₁-C₆, alcényle en C₃-C₆, alcynyle en C₃-C₆, tandis que ces restes peuvent chacun porter un à cinq atomes d'halogène et/ou un à deux des groupes suivants: alcoxy en C₁-C₆, alcényloxy en C₃-C₆, alcynyloxy en C₃-C₆, alkylthio en C₁-C₆, alcénylthio en C₃-C₆, alcynylthio en C₃-C₆, halogénoalcoxy en C₁-C₆, cyano, alkyl(C₁-C₆)carbonyle, alcényl(C₃-C₆)carbonyle, alcynyl(C₃-C₆)carbonyle, alcoxy(C₁-C₆)carbonyle, alcényloxy(C₃-C₆)carbonyle, alcynyloxy(C₃-C₆)carbonyle, bis-dialkyl(C₁-C₆)amino, cycloalkyle en C₃-C₆, phényle éventuellement substitué;
cycloalkyle en C₃-C₆ éventuellement substitué;
phényle éventuellement substitue;
ou R⁶ forme conjointement avec R⁷ une chaîne alkylène en C₄-C₇ éventuellement substituée, dans laquelle un groupe CH₂ peut être remplacé par de l'oxygène, du soufre ou -NH;
ou R¹ représente un groupe 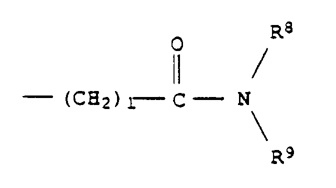 dans lequel R⁸ et R⁹ désignent l'hydrogène ou un groupe alkyle en C₁-C₆, alcényle en C₃-C₆ ou alcynyle en C₃-C₆ et l peut valoir 1, 2, 3 ou 4;
ou R¹ représente un groupe
-(CH₂)ₗ-S(=O)ₖ-R¹⁰
dans lequel R¹⁰ désigne un groupe alkyle en C₁-C₆, alcényle en C₃-C₆ ou alcynyle en C₃-C₆, l vaut 1, 2, 3 ou 4 et k vaut 0, 1 ou 2;
ou R¹ représente un reste
-HN-SO₂-R¹¹,
dans lequel R¹¹ désigne le reste alkyle en C₁-C₆ ou phényle, qui peuvent à leur tour porter un à quatre des substituants suivants: halogène, nitro, cyano, alkyle en C₁-C₆;
ou R¹ représente un reste OR⁵, dans lequel
R⁵ désigne un hétérocycle aromatique à 5 chaînons, éventuellement substitué, lié par un atome d'azote et ayant un à quatre atomes d'azote dans le cycle;
ou R¹ représente un reste 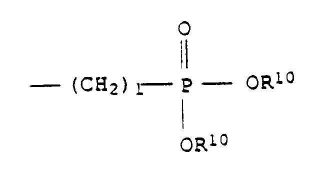 où R¹⁰ et l ont la signification susdite;
l'expression éventuellement substitué dans les définitions qui précèdent signifiant que les groupes ainsi qualifiés peuvent porter un ou plusieurs des substituants suivants: halogène, nitro, cyano, alkyle en C₁-C₆, halogénoalkyle en C₁-C₆, alcoxy en C₁-C₆, alkylthio en C₁-C₆;
R², R³ représentent un groupe alkyle en C₁-C₄, halogénoalkyle en C₁-C₄, alcoxy en C₁-C₄, halogénoalcoxy en C₁-C₄ et/ou alkylthio en C₁-C₄;
R⁴ l'hydrogène, un halogène ou un groupe nitro, alkyle en C₁-C₄, cyano ou halogénoalkyle en C₁-C₄;
X un atome d'oxygène ou de soufre;
Z l'azote ou le groupe méthine.

2. Dérivés d'acide salicylique de formule I selon la revendication 1, dans laquelle n vaut 1, 2 ou 3 et les substituants désignent les atomes ou groupes suivants:
R halogène, alkyle en C₁-C₄;
R¹ un reste ONR⁶R⁷, dans lequel R⁶ et R⁷ désignent l'hydrogène ou un groupe alkyle en C₁-C₄ ou R⁶ forme avec R⁷ une chaîne alkylène en C₄-C₆, dans laquelle un groupe CH₂ peut être remplacé par de l'oxygène ou du soufre;
un reste OR⁵, dans lequel R⁵ est un groupe 1-pyrazolyle ou 1-imidazolyle;
NHSO₂CH₃, NHSO₂C₆H₅;
R², R³ méthoxy;
R⁴ hydrogène;
X oxygène ou soufre;
Z azote ou le groupe méthine.

3. Dérivés d'acide salicylique de formule 1 selon la revendication 1, dans laquelle R¹ représente O-N(CH₃)₂, X désigne l'oxygène, Y désigne l'azote, Z est le groupe méthine, R² et R³ représentent le groupe méthoxy et (R) est 4-Cl, 4-Br ou 2-CH₃.

4. Agent herbicide, contenant un composé de formule I selon la revendication 1 et des additifs inertes classiques.

5. Procédé pour lutter contre la croissance de plantes indésirables, caractérisé en ce qu'on traite les plantes indésirables et/ou leur biotope avec une quantité efficace du point de vue herbicide d'un composé I selon la revendication 1.

6. Utilisation de dérivés d'acide salicylique et de leurs analogues soufrés de formule I selon la revendication 1 en tant qu'herbicides.

7. Agent pour agir sur la croissance des plantes, contenant un dérivé d'acide salicylique de formule I selon la revendication 1 et des additifs inertes classiques.

8. Procédé pour la régulation de la croissance des plantes, caractérisé en ce qu'on fait agir une quantité efficace à des fins de régulation d'un dérivé d'acide salicylique de formule générale I selon la revendication 1 sur les graines, les plantes et/ou leur biotope.

9. Agent fongicide et/ou inhibant la nitrification, contenant un dérivé d'acide salicylique de formule I selon la revendication 1 et des additifs inertes classiques.

## Revendications (Revendications pour l'(les) Etat(s) contractant(s) suivant(s): ES)

1. Agent herbicide, contenant des dérivés d'acide salicylique et leurs analogues soufrés de formule I, 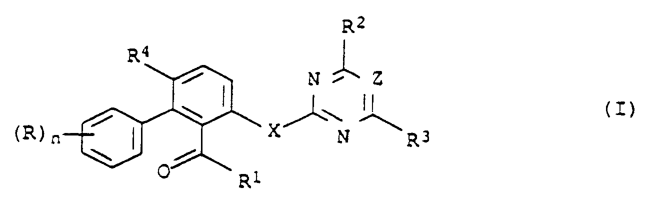 où les substituants désignent les atomes ou groupes suivants:
R halogène, alkyle en C₁-C₄, halogénoalkyle en C₁-C₄, alcoxy en C₁-C₄, halogénoalcoxy en C₁-C₄ et/ou alkylthio en C₁-C₄;
n un nombre de 1 à 3 et, dans le cas d'un halogène comme substituant, un nombre de 1 à 5,
R¹ un reste 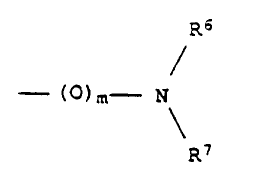 dans lequel m vaut 0 ou 1 et où R⁶ et R⁷ désignent les atomes ou groupes suivants:
hydrogène,
alkyle en C₁-C₆, alcényle en C₃-C₆, alcynyle en C₃-C₆, tandis que ces restes peuvent chacun porter un à cinq atomes d'halogène et/ou un à deux des groupes suivants: alcoxy en C₁-C₆, alcényloxy en C₃-C₆, alcynyloxy en C₃-C₆, alkylthio en C₁-C₆, alcénylthio en C₃-C₆, alcynylthio en C₃-C₆, halogénoalcoxy en C₁-C₆, cyano, alkyl(C₁-C₆)carbonyle, alcényl(C₃-C₆)carbonyle, alcynyl(C₃-C₆)carbonyle, alcoxy(C₁-C₆)carbonyle, alcényloxy(C₃-C₆)carbonyle, alcynyloxy(C₃-C₆)carbonyle, bis-dialkyl(C₁-C₆)amino, cycloalkyle en C₃-C₆, phényle éventuellement substitué;
cycloalkyle en C₃-C₆ éventuellement substitué;
phényle éventuellement substitué;
ou R⁶ forme conjointement avec R⁷ une chaîne alkylène en C₄-C₇ éventuellement substituée, dans laquelle un groupe CH₂ peut être remplacé par de l'oxygène, du soufre ou -NH;
ou R¹ représente un groupe 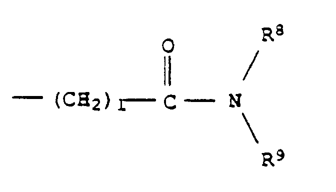 dans lequel R⁸ et R⁹ désignent l'hydrogène ou un groupe alkyle en C₁-C₆, alcényle en C₃-C₆ ou alcynyle en C₃-C₆ et l peut valoir 1, 2, 3 ou 4;
ou R¹ représente un groupe
-(CH₂)ₗ-S(=O)ₖ-R¹⁰
dans lequel R¹⁰ désigne un groupe alkyle en C₁-C₆, alcényle en C₃-C₆ ou alcynyle en C₃-C₆, l vaut 1, 2, 3 ou 4 et k vaut 0, 1 ou 2;
ou R¹ représente un reste
-HN-SO₂-R¹¹,
dans lequel R¹¹ désigne le reste alkyle en C₁-C₆ ou phényle, qui peuvent à leur tour porter un à quatre des substituants suivants: halogène, nitro, cyano, alkyle en C₁-C₆;
ou R¹ représente un reste OR⁵, dans lequel
R⁵ désigne un hétérocycle aromatique à 5 chaînons, éventuellement substitué, lié par un atome d'azote et ayant un à quatre atomes d'azote dans le cycle;
ou R¹ représente un reste 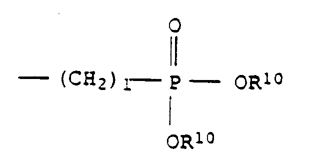 où R¹⁰ et l ont la signification susdite;
l'expression éventuellement substitué dans les définitions qui précèdent signifiant que les groupes ainsi qualifiés peuvent porter un ou plusieurs des substituants suivants: halogène, nitro, cyano, alkyle en C₁-C₆, halogénoalkyle en C₁-C₆, alcoxy en C₁-C₆, alkylthio en C₁-C₆;
R², R³ représentent un groupe alkyle en C₁-C₄, halogénoalkyle en C₁-C₄, alcoxy en C₁-C₄, halogénoalcoxy en C₁-C₄ et/ou alkylthio en C₁-C₄;
R⁴ l'hydrogène, un halogène ou un groupe nitro, alkyle en C₁-C₄, cyano ou halogénoalkyle en C₁-C₄;
X un atome d'oxygène ou de soufre;
Z l'azote ou le groupe méthine.

2. Agent herbicide, contenant des dérivés d'acide salicylique de formule I selon la revendication 1, dans laquelle n vaut 1, 2 ou 3 et les substituants désignent les atomes ou groupes suivants:
R halogène, alkyle en C₁-C₄;
R¹ un reste ONR⁶R⁷, dans lequel R⁶ et R⁷ désignent l'hydrogène ou un groupe alkyle en C₁-C₄ ou R⁶ forme avec R⁷ une chaîne alkylène en C₄-C₆, dans laquelle un groupe CH₂ peut être remplacé par de l'oxygène ou du soufre;
un reste OR⁵, dans lequel R⁵ est un groupe 1-pyrazolyle ou 1-imidazolyle;
NHSO₂CH₃, NHSO₂C₆H₅;
R², R³ méthoxy;
R⁴ hydrogène;
X oxygène ou soufre;
Z azote ou le groupe méthine.

3. Agent, contenant des dérivés d'acide salicylique de formule I selon la revendication 1, dans laquelle R¹ représente O-N(CH₃)₂, X désigne l'oxygène, Y désigne l'azote, Z est le groupe méthine, R² et R³ représentent le groupe méthoxy et (R) est 4-Cl, 4-Br ou 2-CH₃.

4. Agent herbicide, contenant un composé de formule I selon la revendication 1 et des additifs inertes classiques.

5. Procédé pour lutter contre la croissance de plantes indésirables, caractérisé en ce qu'on traite les plantes indésirables et/ou leur biotope avec une quantité efficace du point de vue herbicide d'un composé I selon la revendication 1.

6. Utilisation de dérivés d'acide salicylique et de leurs analogues soufrés de formule I selon la revendication 1 en tant qu'herbicides.

7. Agent pour agir sur la croissance des plantes, contenant un dérivé d'acide salicylique de formule I selon la revendication 1 et des additifs inertes classiques.

8. Procédé pour la régulation de la croissance des plantes, caractérisé en ce qu'on fait agir une quantité efficace à des fins de régulation d'un dérivé d'acide salicylique de formule générale I selon la revendication 1 sur les graines, les plantes et/ou leur biotope.

9. Agent fongicide et/ou inhibant la nitrification, contenant un dérivé d'acide salicylique de formule I selon la revendication 1 et des additifs inertes classiques.
